(19) Europäisches Patentamt
European Patent Office
Office européen des brevets

(11) Veröffentlichungsnummer: **0 480 242 A2**

(12) **EUROPÄISCHE PATENTANMELDUNG**

(21) Anmeldenummer: **91116292.3**

(22) Anmeldetag: **25.09.91**

(51) Int. Cl.[5]: **C07C 45/68**, C07C 47/232, C07C 47/24

(30) Priorität: **06.10.90 DE 4031723**

(43) Veröffentlichungstag der Anmeldung: **15.04.92 Patentblatt 92/16**

(84) Benannte Vertragsstaaten: **BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Isak, Heinz, Dr.**
**Schoenfelderstrasse 3**
**W-6704 Mutterstadt(DE)**
Erfinder: **Goetz, Norbert, Dr.**
**Schoefferstrasse 25**
**W-6520 Worms 1(DE)**
Erfinder: **Kuekenhoehner, Thomas, Dr.**
**Seidelstrasse 2**
**W-6710 Frankenthal(DE)**

(54) **Verfahren zur Herstellung von alpha,beta-ungesättigten Carbonylverbindungen.**

(57) Verfahren zur Herstellung von $\alpha,\beta$-ungesättigten Carbonylverbindungen der allgemeinen Formel I

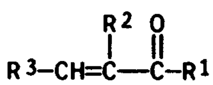

(I),

in der

- $R^1$ Wasserstoff, $C_1$- bis $C_{10}$-Alkyl, $C_1$- bis $C_{10}$-Alkoxy oder Aryloxy,
- $R^2$ gegebenenfalls durch $C_1$- bis $C_4$-Alkyl, $C_1$- bis $C_4$-Alkoxy, Trifluormethyl und/oder Halogen substituiertes Aryl und
- $R^3$ Tetrahydrofuranyl oder durch $C_1$- bis $C_4$-Alkyl, $C_1$- bis $C_4$-Alkoxy, Trifluormethyl und/oder Halogen substituiertes Aryl

bedeuten, dadurch gekennzeichnet, daß man 3-Aminoprop-2-en-1-one der allgemeinen Formel II

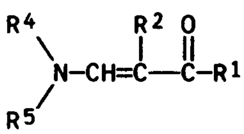

(II),

in der $R^1$ und $R^2$ die obengenannten Bedeutungen haben, und

$R^4$, $R^5$ unabhängig voneinander Wasserstoff, $C_1$- bis $C_{10}$-Alkyl oder Aryl

bedeuten, mit einem Magnesiumhalogenid der allgemeinen Formel III

$R^3$-Mg-Y (III),

in der Y für Halogen steht, bei Temperaturen von (-20) bis 100 °C umsetzt, sowie neue $\alpha,\beta$-ungesättigte Carbonylverbindungen und neue 3-Aminoprop-2-en-1-one.



Rank Xerox (UK) Business Services

Die vorliegende Erfindung betrifft ein neues Verfahren zur Herstellung von α,β-ungesättigten Carbonylverbindungen sowie neue 3-Aminoprop-2-en-1-one.

Aus J. Amer. Chem. Soc. 71, 2671-2676 (1949) ist bekannt, daß Methylmagnesiumiodid mit Dimethylamino-methylvinylphenylketon in Diethylether zu Phenyl-1,2-dimethylvinylketon abreagiert.

Aus Helv. Chim. Acta (52), 2465-2472 (1969) und C.R. Acad. Sci. C. 278, 1113-1116 (1974) ist die Umsetzung von Methyl-phenyl-aminovinyl-alkylketon mit Alkylhalogeniden in schlechten Ausbeuten (20 %) zu ungesättigte Ketone bekannt.

Aus Chem. Ber., 1359-1366 (1958) ist bekannt, daß Zimtaldehyde durch Reaktion von [N-Methylanilino]-prop-1-en-3-al mit Phenylmagnesiumbromid in 60 %iger Ausbeute entsteht.

Aus der DE-A-34 37 238 und der DE-A-35 36 117 ist die Herstellung von α,β-ungesättigten Carbonylverbindungen durch Kondensationsreaktion von Aldehyden mit Ketonen, speziell Aceton, unter Basenkatalyse bekannt. Hierbei wurden α,β- und β,γ-Isomerengemische erhalten, die nur unter erheblichem Aufwand getrennt werden können.

Der vorliegenden Erfindung lag daher die Aufgabe zugrunde, den zuvorgenannten Nachteilen abzuhelfen.

Demgemäß wird ein neues und verbessertes verfahren zur Herstellung von α,β-ungesättigten Carbonylverbindungen der allgemeinen Formel I

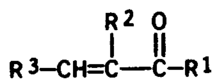

(I),

in der

- $R^1$ Wasserstoff, $C_1$- bis $C_{10}$-Alkyl, $C_1$- bis $C_{10}$-Alkoxy oder Aryloxy,
- $R^2$ gegebenenfalls durch $C_1$- bis $C_4$-Alkyl, $C_1$- bis $C_4$-Alkoxy, Trifluormethyl und/oder Halogen substituiertes Aryl und
- $R^3$ Tetrahydrofuranyl oder durch $C_1$- bis $C_4$-Alkyl, $C_1$- bis $C_4$-Alkoxy, Trifluormethyl und/oder Halogen substituiertes Aryl

bedeuten, gefunden, welches dadurch gekennzeichnet ist, daß man 3-Aminoprop-2-en-1-one der allgemeinen Formel II

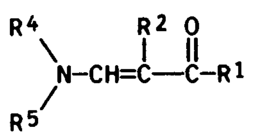

(II),

in der $R^1$ und $R^2$ die obengenannten Bedeutungen haben, und

- $R^4$, $R^5$ unabhängig voneinander Wasserstoff, $C_1$- bis $C_{10}$-Alkyl oder Aryl

bedeuten, mit einem Magnesiumhalogenid der allgemeinen Formel III

$R^3$-Mg-Y (III),

in der Y für Halogen steht, bei Temperaturen von (-20) bis 100 °C umsetzt, sowie neue 3-Aminoprop-2-en-1-one der allgemeinen Formel II'

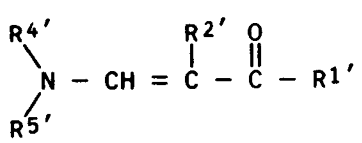

(II')

in der

- $R^{1'}$ Wasserstoff, $C_1$- bis $C_{10}$-Alkyl, $C_1$- bis $C_{10}$-Alkoxy oder Aryloxy,
- $R^{2'}$ gegebenenfalls durch $C_1$- bis $C_4$-Alkyl, $C_1$- bis $C_4$-Alkoxy, Trifluormethyl und/oder Halogen substituiertes Aryl und
- $R^{4'}$ $R^{5'}$ $C_1$- bis $C_{10}$-Alkyl

bedeuten, mit der Maßgabe, dap $R^{1'}$ nicht für Methyl steht, wenn $R^{2'}$ für Phenyl steht.

Das erfindungsgemäße Verfahren läßt sich wie folgt durchführen:

Zu einer Lösung bzw. Suspension oder Aufschlämmung eines Magnesiumhalogenids der Formel III wird bei Temperaturen von (-20) bis 100 ° C, vorzugsweise (-10) bis 50 ° C, besonders bevorzugt bei 0 bis 20 ° C ein 3-Aminoprop-2-en-1-on der Formel II, zugegeben.

Das Molverhältnis von III zu II beträgt zwischen 0,8:1 und 20:1, bevorzugt 1:1 bis 5:1, besonders bevorzugt 1:1 bis 1,2:1.

Als Lösungsmittel eignen sich alle für Grignardreaktionen üblichen Lösungsmittel beispielsweise dipolare, aprotische organische Lösungsmittel, z.B. Ether, wie Diethylether, Methyl-tert.-butylether, Tetrahydrofuran, Dioxan und Dimethylglykolether; oder Amine, wie Triethylamin, oder apolare, aprotische organische Lösungsmittel, z.B. aliphatische Kohlenwasserstoffe, wie Cyclohexan oder aromatische Kohlenwasserstoffe, wie Benzol, Toluol als Gemisch mit dipolaren, aprotischen Lösungsmitteln. Bevorzugt werden acylische und cyclische Ether, wie Diethylether, Methyl-tert.-butylether, Tetrahydrofuran, Dioxan und Dimethylglykolether, besonders bevorzugt werden cyclische Ether wie Tetrahydrofuran und Dioxan.

Die Lösungsmittel können Wasser enthalten, werden jedoch bevorzugt mit 0 bis 5 Gew.% Wasser, besonders bevorzugt wasserfrei eingesetzt.

Als Magnesiumhalogenide der Formel III eignen sich die Fluoride, Chloride, Bromide und Jodide, bevorzugt werden Chloride und Bromide, besonders bevorzugt Chloride.

Die Magnesiumhalogenide der Formel III lassen sich wie üblich durch Umsetzung der entsprechenden Halogenide mit Magnesium in einem der für Grignardreaktionen üblichen Lösungsmittel herstellen.

Die Substituenten $R^1$, $R^2$, $R^3$, $R^4$ und $R^5$ in den Verbindungen I, II und III haben folgende Bedeutungen:

$R^1$

- Wasserstoff,
- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl, n-Propyl, iso-Propyl, n-Butyl, iso-Butyl, sec.-Butyl und tert.-Butyl,
- $C_1$- bis $C_{10}$-Alkoxy, bevorzugt $C_1$- bis $C_8$-Alkoxy, besonders bevorzugt $C_1$- bis $C_4$-Alkoxy wie Methoxy, Ethoxy, n-Propoxy, iso-Propoxy, n-Butoxy, iso-Butoxy, sec.-Butoxy und tert.-Butoxy,
- Aryloxy, bevorzugt Phenoxy,

$R^2$

- Aryl wie Phenyl, 1-Naphthyl und 2-Naphthyl, bevorzugt Phenyl,
- ein- bis dreifach durch $C_1$- bis $C_4$-Alkyl substituiertes Aryl, bevorzugt ein- bis dreifach durch $C_1$- bis $C_4$-Alkyl substituiertes Phenyl wie 2-Methyl-phenyl, 2,4-Dimethyl-phenyl, 2,4-Diisopropyl-phenyl und 2,4,6-Trimethyl-phenyl,
- ein- bis dreifach durch $C_1$- bis $C_4$-Alkoxy substituiertes Aryl, bevorzugt ein- bis dreifach durch $C_1$- bis $C_4$-Alkoxy substituiertes Phenyl wie 2,4-Dimethoxyphenyl, 4-Methoxyphenyl, 2,4,6-Trimethoxyphenyl, 2,4-Diethoxyphenyl und 4-Propyloxyphenyl,
- ein- bis dreifach durch Trifluormethyl substituiertes Aryl, bevorzugt ein- bis dreifach durch Trifluormethyl substituiertes Phenyl wie 4-Trifluormethylphenyl, 3-Chlor-4-trifluormethylphenyl und 2-Trifluormethyl-4-methylphenyl,
- ein- bis dreifach durch Halogen substituiertes Aryl, bevorzugt ein- bis dreifach durch Halogen substituiertes Phenyl wie 2-Chlorphenyl, 2,4-Dichlorphenyl, 2-Fluorphenyl, 2,4-Difluorphenyl und 4-Fluorphenyl,
- ein- bis dreifach durch $C_1$- bis $C_4$-Alkyl und $C_1$- bis $C_4$-Alkoxy substituiertes Aryl, bevorzugt ein- bis dreifach durch $C_1$- bis $C_4$-Alkyl und $C_1$- bis $C_4$-Alkoxy substituiertes Phenyl wie 4-Methoxyphenyl, 2-Isopropylphenyl, 2-Methoxy-3-methylphenyl und 4-Methoxy-2,6-dimethylphenyl,
- ein- bis dreifach durch $C_1$- bis $C_4$-Alkyl und Trifluormethyl substituiertes Aryl, bevorzugt ein- bis dreifach durch $C_1$- bis $C_4$-Alkyl und Trifluormethyl substituiertes Phenyl wie 4-Trifluormethyl-2-methylphenyl und 4-Trifluormethyl-2-isopropylphenyl,
- ein- bis dreifach durch $C_1$- bis $C_4$-Alkyl und Halogen substituiertes Aryl, bevorzugt ein- bis dreifach durch $C_1$- bis $C_4$-Alkyl und Halogen substituiertes Phenyl wie 2-Methyl-4-chlor-phenyl, 2-Chlor-4-methyl-phenyl, 2-Fluor-4-methyl-phenyl und 2-Methyl-4-fluorphenyl,
- ein- bis dreifach durch $C_1$- bis $C_4$-Alkoxy und Trifluormethyl substituiertes Aryl, bevorzugt ein- bis dreifach durch $C_1$- bis $C_4$-Alkoxy und Trifluormethyl substituiertes Phenyl wie 2,6-Dimethoxy-4-trifluormethylphenyl und 3-Ethoxy-5-trifluormethylphenyl,

- ein- bis dreifach durch $C_1$- bis $C_4$-Alkoxy und Halogen substituiertes Aryl, bevorzugt ein- bis dreifach durch $C_1$- bis $C_4$-Alkoxy und Halogen substituiertes Phenyl,
- ein- bis dreifach durch Trifluormethyl und Halogen substituiertes Aryl, bevorzugt ein- bis dreifach durch Trifluormethyl und Halogen $C_4$-Alkoxy substituiertes Phenyl wie 2-Fluor-4-trifluormethylphenyl und 2-Chlor-4-trifluormethylphenyl,

$R^3$

- Tetrahydrofuranyl,
- bevorzugt ein- bis dreifach durch $C_1$- bis $C_4$-Alkyl substituiertes Phenyl wie 2-Methyl-phenyl, 2,4-Dimethyl-phenyl, 2,4-Diisopropyl-phenyl und 2,4,6-Trimethylphenyl,
- ein- bis dreifach durch $C_1$- bis $C_4$-Alkoxy substituiertes Aryl, bevorzugt ein- bis dreifach durch $C_1$- bis $C_4$-Alkoxy substituiertes Phenyl wie 2,4-Dimethoxy-phenyl, 2,4,6-Trimethoxyphenyl und 2,4-Triethoxyphenyl,
- ein- bis dreifach durch Trifluormethyl substituiertes Aryl, bevorzugt ein- bis dreifach durch Trifluormethyl substituiertes Phenyl wie 4-Trifluormethylphenyl und 2-Chlor-4-trifluormethylphenyl,
- ein- bis dreifach durch Halogen substituiertes Aryl, bevorzugt ein- bis dreifach durch Halogen substituiertes Phenyl wie 2-Chlorphenyl, 2,4-Dichlorphenyl, 2-Fluorphenyl, 2,4-Difluorphenyl und 4-Fluorphenyl,
- ein- bis dreifach durch $C_1$- bis $C_4$-Alkyl und $C_1$- bis $C_4$-Alkoxy substituiertes Aryl, bevorzugt ein- bis dreifach durch $C_1$- bis $C_4$-Alkyl und $C_1$- bis $C_4$-Alkoxy substituiertes Phenyl wie 4-Methoxy-2-isopropyl-phenyl, 2-Methoxy-3-methyl-phenyl und 4-Methoxy-2,6-dimethylphenyl,
- ein- bis dreifach durch $C_1$- bis $C_4$-Alkyl und Trifluormethyl substituiertes Aryl, bevorzugt ein- bis dreifach durch $C_1$- bis $C_4$-Alkyl und Trifluormethyl wie 4-Trifluormethyl-2-isopropylphenyl und 4-Trifluormethyl-2-methylphenyl,
- ein- bis dreifach durch $C_1$- bis $C_4$-Alkyl und Halogen substituiertes Aryl, bevorzugt ein- bis dreifach durch $C_1$- bis $C_4$-Alkyl und Halogen substituiertes Phenyl wie 2-Methyl-4-chlorphenyl, 2-Chlor-4-methylphenyl, 2-Fluor-4-methylphenyl und 2-Methyl-4-fluorphenyl,
- ein- bis dreifach durch $C_1$- bis $C_4$-Alkoxy und Trifluormethyl substituiertes Aryl, bevorzugt ein- bis dreifach durch $C_1$- bis $C_4$-Alkoxy und Trifluormethyl wie 2-Methoxy-4-trifluormethylphenyl und 2,6-Dimethyl-4-fluorphenyl,
- ein- bis dreifach durch $C_1$- bis $C_4$-Alkoxy und Halogen substituiertes Aryl, bevorzugt ein- bis dreifach durch $C_1$- bis $C_4$-Alkoxy und Halogen substituiertes Phenyl wie 2,6-Dichlor-4-ethoxyphenyl und 2-Chlor-4-methylphenyl und 2-Fluor-4-methoxyphenyl,
- ein- bis dreifach durch Trifluormethyl und Halogen substi-tuiertes Aryl, bevorzugt ein- bis dreifach durch Trifluormethyl und Halogen substituiertes Phenyl wie 2-Fluor-4-trifluormethylphenyl, 2,6-Dichlor-4-trifluormethylphenyl und 4-Cl-2-trifluormethylphenyl,

$R^4$, $R^5$

- unabhängig voneinander
- Wasserstoff,
- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl, n-Propyl, iso-Propyl, n-Butyl, iso-Butyl, sec.-Butyl und tert.-Butyl,
- Aryl wie Phenyl.

Unter den nach dem neuen Verfahren herstellbaren Verbindungen I seien beispielsweise genannt:

2-Phenyl-but-2-en-1-yl
2-(4-Chlor-phenyl)-but-2-en-1-al
2-(2,4-Dichlor-phenyl)-but-2-en-1-al
2-(2-Methyl-phenyl)-pent-3-en-2-on
2-(2,4-Dichlor-phenyl)-pent-3-en-2-on
2-(4-Trifluormethyl-phenyl)-pent-3-en-2-on
2-(2-Methylphenyl)-but-2-en-methylester
2-(2,4-Dichlor-phenyl)-but-2-en-methylester
2-(2-Methylphenyl)-but-2-en-ethylester
2-(2,4-Dichlor-phenyl)-but-2-en-ethylester
2-(2-Chlor-phenyl)but-2-en-propylester
2-(Methyl-methylen)-cyclohexan-1-on
2-(Methyl-methylen)-6-methyl-cyclohexan-1-on

2-(dimethyl-methylen)-cyclohexan-1-on
2-(4-Fluor-phenyl)-3-(2,4-dimethyl-phenyl)-acrolein
2-Phenyl-3-(4-methyl-phenyl)-acrolein
2-Phenyl-3-(3,4-dichlor-phenyl)-acrolein
2-Phenyl-3-(2,4-dichlor-phenyl)-acrolein
2-Phenyl-3-(2,4,6-trichlor-phenyl)-acrolein
2-Phenyl-3-(2,4,6-trimethyl-phenyl)-acrolein
2-Phenyl-3-(3,4,5-trichlor-phenyl)-acrolein
2-(4-Fluor-phenyl)-3-(2-methyl-phenyl)-acrolein
2-Phenyl-3-(2-trifluormethyl-phenyl)-acrolein
2-(4-Chlor-phenyl)-3-(2-methoxy-phenyl)-acrolein
2-(4-Fluor-phenyl)-3-(4-trifluor-phenyl)-acrolein
2-(4-Bromphenyl)-3-(4-chlor-phenyl)-acrolein

Die Substituenten $R^{1'}$, $R^{2'}$, $R^{4'}$ und $R^{5'}$ der Verbindungen II' haben folgende Bedeutungen:

$R^{1'}$

- Wasserstoff,
- $C_1$- $C_{10}$-Alkyl, bevorzugt $C_1$- bis $C_8$-Alkyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_1$- bis $C_{10}$-Alkoxy, bevorzugt $C_1$- bis $C_8$-Alkoxy, besonders bevorzugt $C_1$- bis $C_4$-Alkoxy wie Methoxy, Ethoxy, n-Propoxy, iso-Propoxy, n-Butoxy, iso-Butoxy, sec.-Butoxy und tert.-Butoxy,
- Aryloxy, bevorzugt Phenoxy,

$R^{2'}$

- Aryl wie Phenyl, 1-Naphthyl und 2-Naphthyl, bevorzugt Phenyl,
- ein- bis dreifach durch $C_1$- bis $C_4$-Alkyl substituiertes Aryl, bevorzugt ein- bis dreifach durch $C_1$- bis $C_4$-Alkyl substituiertes Phenyl wie 2-Methyl-phenyl, 2,4-Dimethyl-phenyl, 2,4-Diisopropyl-phenyl und 2,4,6-Trimethyl-phenyl,
- ein- bis dreifach durch $C_1$- bis $C_4$-Alkoxy substituiertes Aryl, bevorzugt ein- bis dreifach durch $C_1$- bis $C_4$-Alkoxy substituiertes Phenyl wie 2,4-Dimethoxyphenyl, 4-Methoxy-2,4,6-trimethoxyphenyl, 2,4-Diethoxyphenyl und 4-Propyloxyphenyl,
- ein- bis dreifach durch Trifluormethyl substituiertes Aryl, bevorzugt ein- bis dreifach durch Trifluormethyl substituiertes Phenyl wie 4-Trifluormethylphenyl, 3-Chlor-4-trifluormethylphenyl und 2-Trifluormethyl-4-methylphenyl,
- ein- bis dreifach durch Halogen substituiertes Aryl, bevorzugt ein- bis dreifach durch Halogen substituiertes Phenyl wie 2-Chlorphenyl, 2,4-Dichlorphenyl, 2-Fluorphenyl, 2,4-Difluorphenyl und 4-Fluorphenyl,
- ein- bis dreifach durch $C_1$- bis $C_4$-Alkyl und $C_1$- bis $C_4$-Alkoxy substituiertes Aryl, bevorzugt ein- bis dreifach durch $C_1$- bis $C_4$-Alkyl und $C_1$- bis $C_4$-Alkoxy substituiertes Phenyl wie 4-Methoxyphenyl, 2-Isopropylphenyl, 2-Methoxy-3-methylphenyl und 4-Methoxy-2,6-dimethylphenyl,
- ein- bis dreifach durch $C_1$- bis $C_4$-Alkyl und Trifluormethyl substituiertes Aryl, bevorzugt ein- bis dreifach durch $C_1$– bis $C_4$-Alkyl und Trifluormethyl substituiertes Phenyl wie 4-Trifluormethyl-2-methylphenyl und 4-Trifluormethyl-2-isopropylphenyl,
- ein- bis dreifach durch $C_1$- bis $C_4$-Alkyl und Halogen substituiertes Aryl, bevorzugt ein- bis dreifach durch $C_1$- bis $C_4$-Alkyl und Halogen substituiertes Phenyl wie 2-Methyl-4-chlor-phenyl, 2-Chlor-4-methyl-phenyl, 2-Fluor-4-methyl-phenyl und 2-Methyl-4-fluorphenyl,
- ein- bis dreifach durch $C_1$- bis $C_4$-Alkoxy und Trifluormethyl substituiertes Aryl, bevorzugt ein- bis dreifach durch $C_1$- bis $C_4$-Alkoxy und Trifluormethyl substituiertes Phenyl wie 2,6-Dimethoxy-4-trifluormethylphenyl und 3-Ethoxy-5-trifluormethylphenyl,
- ein- bis dreifach durch $C_1$- bis $C_4$-Alkoxy und Halogen substituiertes Aryl, bevorzugt ein- bis dreifach durch $C_1$- bis $C_4$-Alkoxy und Halogen substituiertes Phenyl,
- ein- bis dreifach durch Trifluormethyl und Halogen substituiertes Aryl, bevorzugt ein- bis dreifach durch Trifluormethyl und Halogen $C_4$-Alkoxy substituiertes Phenyl wie 2-Fluor-4-trifluormethylphenyl und 2-Chlor-4-trifluormethylphenyl,

$R^{4'}$, $R^{5'}$

- unabhängig voneinander
- Wasserstoff,

- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl, n-Propyl, iso-Propyl, n-Butyl, iso-Butyl, sec.-Butyl und tert.-Butyl,
- Aryloxy, bevorzugt Phenoxy.

Unter den neuen Verbindungen II' sind die folgenden besonders bevorzugt:

| $R^{1'}$ | $R^{2'}$ | $R^{4'}$ | $R^{5'}$ |
|---|---|---|---|
| Wasserstoff | Phenyl | Methyl | Methyl |
| Methyl | 2-Methylphenyl | Methyl | Methyl |
| Methoxy | 2-Methylphenyl | Methyl | Methyl |
| Wasserstoff | 4-Fluorphenyl | Methyl | Methyl |
| Wasserstoff | 4-Chlorphenyl | Methyl | Methyl |

Die gemäß dem erfindungsgemäßen Verfahren herstellbaren Verbindungen I und die neuen Verbindungen I' eignen sich als Zwischenprodukte für Wirkstoffe im Pflanzenschutz (DE-A-34 37 238, DE-A-35 36 117 und DE-A-36 01 066).

Beispiele

Beispiel 1

Herstellung von 3-Phenyl-but-2-en-1-al

2,16 mol einer 1,5-molaren Methylmagnesiumchloridlösung in Tetrahydrofuran wird bei 15 bis 20 °C vorgelegt. Unter Kühlung erfolgt innerhalb von 3 Stunden die Zugabe von dem 3-Dimethylamino-3-phenyl-prop-2-en-1-al in 500 ml Tetrahydrofuran. Der Austrag wird in 3 Liter einer gesättigten $NH_4Cl$-Lösung eingerührt und durch Zugabe von Wasser gelöst. Nach Phasentrennung und fraktionierter Destillation erhält man 210 g (93 %) 3-Phenyl-but-2-en-1-al Sdp. 80 bis 90 °C; 0,35 Torr.

Analog Beispiel 1 wurden die in der folgenden Tabelle aufgeführten Verbindungen hergestellt:

Tabelle

| Verbindung Nr. | $R^1$ | $R^2$ | $R^3$ | Sdp. [°C/mbar] | Fp. [°C] |
|---|---|---|---|---|---|
| 2 | H | 4-Fluorphenyl | 2,4-Dimethylphenyl | 135/0,1 | - |
| 3 | H | Phenyl | 4-Methylphenyl | 134/0,2 | - |
| 4 | H | Phenyl | 3,4-Dichlorphenyl | - | 124 |
| 5 | H | Phenyl | 2,4-Dichlorphenyl | - | 98 |
| 6 | H | Phenyl | 2,4,6-Trimethylphenyl | - | 110 |
| 7 | H | Phenyl | 3,4,5-Trichlorphenyl | - | 165 |
| 8 | H | 4-Fluorphenyl | 2-Methylphenyl | 135/0,3 | 75 |
| 9 | H | Phenyl | 2-Trifluormethylphenyl | - | 64 |
| 10 | H | 4-Chlorphenyl | 2-Methoxyphenyl | 175/0,4 | - |
| 11 | H | 4-Fluorphenyl | 4-Trifluormethylphenyl | 142/0,25 | 86 |

## Patentansprüche

**1.** Verfahren zur Herstellung von $\alpha,\beta$-ungesättigten Carbonylverbindungen der allgemeinen Formel I

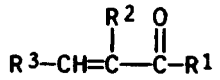

(I),

in der

$R^1$ Wasserstoff, $C_1$- bis $C_{10}$-Alkyl, $C_1$- bis $C_{10}$-Alkoxy oder Aryloxy,

$R^2$ gegebenenfalls durch $C_1$- bis $C_4$-Alkyl, $C_1$- bis $C_4$-Alkoxy, Trifluormethyl und/oder Halogen substituiertes Aryl und

$R^3$ Tetrahydrofuranyl oder durch $C_1$- bis $C_4$-Alkyl, $C_1$- bis $C_4$-Alkoxy, Trifluormethyl und/oder Halogen substituiertes Aryl

bedeuten, dadurch gekennzeichnet, daß man 3-Aminoprop-2-en-1-one der allgemeinen Formel II

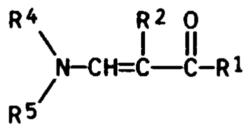

(II),

in der $R^1$ und $R^2$ die obengenannten Bedeutungen haben, und

$R^4$, $R^5$ unabhängig voneinander Wasserstoff, $C_1$- bis $C_{10}$-Alkyl oder Aryl

bedeuten, mit einem Magnesiumhalogenid der allgemeinen Formel III

$R^3$-Mg-Y (III),

in der Y für Halogen steht, bei Temperaturen von (-20) bis 100 ° C umsetzt.

**2.** 3-Aminoprop-2-en-1-one der allgemeinen Formel II'

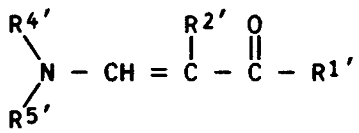

(II')

in der

$R^{1'}$ Wasserstoff, $C_1$- bis $C_{10}$-Alkyl, $C_1$- bis $C_{10}$-Alkoxy oder Aryloxy,

$R^{2'}$ gegebenenfalls durch $C_1$- bis $C_4$-Alkyl, $C_1$- bis $C_4$-Alkoxy, Trifluormethyl und/oder Halogen substituiertes Aryl und

$R^{4'}$, $R^{5'}$ $C_1$- bis $C_{10}$-Alkyl

bedeuten, mit der Maßgabe, daß $R^{1'}$ nicht für Methyl steht, wenn $R^{2'}$ für Phenyl steht.